(19) Europäisches Patentamt
European Patent Office
Office européen des brevets

(11) EP 0 706 791 A1

(12) EUROPEAN PATENT APPLICATION

(43) Date of publication:
**17.04.1996 Bulletin 1996/16**

(51) Int. Cl.⁶: **A61K 7/50**, C11D 1/12

(21) Application number: **95115971.4**

(22) Date of filing: **10.10.1995**

(84) Designated Contracting States:
**DE FR GB**

(30) Priority: **11.10.1994 JP 245178/94**

(71) Applicant: **KAO CORPORATION**
**Chuo-ku, Tokyo (JP)**

(72) Inventors:
- **Matsuo, Takashi**
  **Ichikawa-shi, Chiba (JP)**
- **Mizushima, Hiromoto**
  **Wakayama-shi, Wakayama (JP)**
- **Yahagi, Kazuyuki**
  **Tokyo (JP)**

(74) Representative: **Hansen, Bernd, Dr. Dipl.-Chem. et al**
**Hoffmann, Eitle & Partner,**
**Patentanwälte,**
**Arabellastrasse 4**
**D-81925 München (DE)**

# (54) Detergent composition containing N-alkylcarbanylalcohol sulfate salts and use thereof

(57) It is to provide a detergent composition having an excellent detergency, excellent foaming properties, low irritating properties and a pearly luster, and giving no damage to hair.

The detergent composition comprises a compound represented by the general formula (1):

$$R^1\text{-}NH\text{-}\overset{\overset{O}{\|}}{C}\text{-}CH_2OSO_3K \qquad (1)$$

(wherein $R^1$ stands for a linear or branched alkyl or alkenyl group having 8 to 18 carbon atoms).

EP 0 706 791 A1

## Description

[Field of the Invention]

The present invention relates to a detergent composition having low irritating properties against skin and hair, very high foaming properties, and an appearance of pearly luster.

[Description of Related Art]

High foaming properties, a high detergency, and low irritating properties are required of detergents and particularly of surfactants for use in hair detergents. Conventional detergents and hair detergents in particular mainly comprise an alkyl sulfate or, a salt of a polyoxyethylene alkyl ether sulfate blended therein. Such a surfactant is excellent in foaming properties and detergency, and does not involve a problem of irritating properties when used in such an amount as to be blended in a common detergent. When the amount of such a surfactant to be blended is increased in order to further enhance the foaming properties and detergency of a detergent, however, there is yet room for improvement in the irritating properties thereof.

Further, in order to impart a high-quality touch to such a detergent, development of a detergent having an appearance of pearly luster has recently been under way. A method wherein a platy crystal substance such as distearyl ethylene glycol is dispersed in a liquid for the purpose of providing a pearly luster is adopted in many cases. However, such a pearlizing agent remains in hair to be causative of giving thereto a squeaky feeling during rinsing and a starchy feeling during drying.

In view of the above, there has been an eager demand for development of a detergent having an excellent detergency, excellent foaming properties, low irritating properties and a pearly luster, and giving no damage to hair.

[Summary of the Invention]

Under the foregoing circumstances, the inventors of the present invention have made intensive investigations to find out that a potassium salt of an N-alkylglycolamide sulfate having an amido group derived from a long-chain primary amine or secondary amine and a sulfuric ester group can attain the foregoing purposes. The present invention has been completed based on this finding.

More specifically, the present invention provides a detergent composition characterized by comprising a compound represented by the general formula (1):

$$R^1\text{-NH-}\overset{\overset{\displaystyle O}{\|}}{C}\text{-}CH_2OSO_3K \qquad (1)$$

(wherein $R^1$ stands for a linear or branched alkyl or alkenyl group having 8 to 18 carbon atoms).

The invention provides a detergent composition comprising a compound represented by the general formula (1) and a carrier.

It is preferable that $R^1$ is a linear or branched alkyl or alkenyl group having 10 to 14 carbon atoms, in particular a dodecyl group.

The composition of the invention may further comprises the compound of the below described formula (2), preferably wherein M of the general formula (2) is sodium, ammonium or triethanolammonium.

In addition, the invention provides a method for deterging the skin or hair with the compound as defined above; use of the compound as defined above for deterging skin or hair; a method for manifesting a pearly luster on a detergent composition by using the compound as defined above; and use of the compound as defined above as a manifester of a pearly luster.

In the general formula (1), $R^1$ stands for a linear or branched alkyl or alkenyl group having 8 to 18 carbon atoms, specific examples of which include an octyl group, a decyl group, a dodecyl group, a tetradecyl group, a hexadecyl group, an octadecyl group, an isostearyl group, and an oleyl group. Among them, those having 10 to 14 carbon atoms are preferred, and a dodecyl group is especially preferred because it provides high foaming properties and detergency as well as easily provides an appearance of pearly luster at ordinary temperature.

Although the detergent composition of the present invention can assume a pearly luster by comprising the compound represented by the general formula (1), only the compound represented by the general formula (1) sometimes has a high temperature of manifesting a pearly luster. In view of this, a compound represented by the following general formula (2) is preferably blended in the detergent composition of the present invention since the temperature of manifesting a

pearly luster can be controlled without any adverse effects on the detergency and foaming properties of the composition.

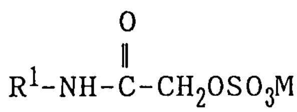

(2)

(wherein $R^1$ stands for the same meaning as defined above; and M stands for an alkali metal except for potassium, an alkaline earth metal, ammonium, an alkanolammonium, an alkylammonium, an alkenylammonium, an alkyl- or alkenyl-substituted pyridinium, or a basic amino acid).

In the general formula (2), M may be selected from the group consisting of alkali metals except for potassium; alkaline earth metals; ammonium; alkanolammoniums, preferably alkanolammoniums having 2 to 9 carbon atoms in total; alkylammoniums or alkenylammoniums having 1 to 22 carbon atoms in total; pyridinium substituted with alkyl or alkenyl having 1 to 18 carbon atoms in total; and basic amino acids. Among them, sodium, ammonium and triethanolammonium are especially preferred.

The compound represented by the general formula (1) according to the present invention can be easily prepared by taking the following steps A and B in this order. On the other hand, the compound represented by the general formula (2) can be easily prepared by taking the following steps A and C in this order.

Step A

A step wherein an aliphatic amine represented by the general formula (3):

$$R^1\text{-}NH_2 \qquad (3)$$

(wherein $R^1$ stands for the same meaning as defined above) is reacted with glycolic acid or an ester thereof represented by the general formula (4):

$$HO\text{-}CH_2CO_2R^2 \qquad (4)$$

(wherein $R^2$ stands for a hydrogen atom or a linear or branched alkyl group having 1 to 4 carbon atoms) to obtain an N-alkylglycolamide represented by the general formula (5):

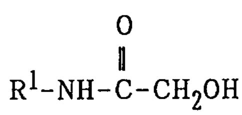

(5)

(wherein $R^1$ stands for the same meaning as defined above).

Step B

A step wherein the N-alkylglycolamide obtained in the step A and represented by the general formula (5) is reacted with a sulfating agent, and then neutralized with a basic substance selected from the group consisting of potassium hydroxide, potassium carbonate and potassium bicarbonate to obtain the compound represented by the aforementioned general formula (1).

Step C

A step wherein the N-alkylglycolamide obtained in the step A and represented by the general formula (5) is reacted with a sulfating agent, and then neutralized with a basic substance other than potassium-containing basic substances to obtain the compound represented by the aforementioned general formula (2).

These steps will now be described in detail.

Step A

This step is a step wherein the aliphatic amine represented by the above-mentioned general formula (3) [hereinafter referred to briefly as the "aliphatic amine (3)"] is reacted with glycolic acid or an ester thereof represented by the above-mentioned general formula (4) [hereinafter referred to briefly as "glycolic acid or an ester thereof (4)"] to obtain the N-alkylglycolamide represented by the above-mentioned general formula (5) [hereinafter referred to briefly as the "N-alkylglycolamide (5)"].

Specific examples of the aliphatic amine (3) include octylamine, decylamine, dodecylamine, tetradecylamine, hexadecylamine, octadecylamine, and isostearylamine, among which decylamine, dodecylamine and tetradecylamine are preferred, and dodecylamine is especially preferred.

Examples of the ester of glycolic acid include methyl, ethyl, propyl, and butyl esters thereof, among which methyl glycolate and ethyl glycolate are preferred particularly by reason of foaming properties and chemical stability.

In this step, the aliphatic amine (3) and glycolic acid or an ester thereof (4), the amount of which is 1 to 2.0 times in terms of mol as much as that of the former, are subjected to dehydration or dealcoholization in the absence of any solvents and, if necessary, in the presence of a basic catalyst such as $CH_3ONa$, KOH or NaOH at 40 to 200°C, preferably at 60 to 150°C, for 1 to 100 hours, preferably for 1 to 20 hours, to obtain the N-alkylglycolamide (5).

The pressure during the reaction is preferably reduced to 760 to 0.1 mmHg because dehydration is necessary.

The N-alkylglycolamide (5) thus obtained can be used as such in the next step, but, if necessary, may be recrystallized from a solvent such as hexane, methanol, ethanol, acetone or chloroform to effect purification thereof.

Step B

This step is a step wherein the N-alkylglycolamide (5) obtained in the step A is reacted with a sulfating agent, and then neutralized with a basic substance selected from the group consisting of potassium hydroxide, potassium carbonate or potassium bicarbonate to obtain the compound represented by the above-mentioned general formula (1) [hereinafter referred to briefly as the "compound (1)"].

Examples of the sulfating agent include chlorosulfonic acid, sulfuric anhydride, fuming sulfuric acid, concentrated sulfuric acid, and sulfamic acid, among which chlorosulfonic acid, sulfuric anhydride and sulfamic acid are preferred from the standpoint of reaction yield.

In this step, the sulfating agent is used in an amount 1 to 1.5 times in terms of mol as much as that of the N-alkylglycolamide (5), and the reaction therebetween is effected in a solvent such as dichloromethane, chloroform, 1,2-dichloroethane, dimethylformamide, dimethyl sulfoxide or dioxane, the amount of which is 0 to 10 times as much as that of the N-alkylglycolamide (5), at -50 to 80°C, preferably at -30 to 50°C, for 1 to 20 hours to obtain the corresponding sulfation product.

Additionally stated, the solvent must be purified into such a state as to be substantially free of water and alcohols. Although this sulfation can also be effected in the absence of any solvents, a countermeasure in an aspect of equipment is necessary because the viscosity of the reaction system is notably increased during the course of the reaction.

On the other hand, when the reaction temperature exceeds 80°C, side reactions such as cleavage of the amido group unfavorably proceed.

After the completion of the sulfation reaction, the solvent used in the reaction is distilled off if necessary. Additionally stated, the solvent may alternatively be distilled off after the following neutralization.

Subsequently, the sulfation product obtained by the foregoing reaction and the aforementioned basic substance, the amount of which is 0.9 to 1.5 times in terms of equivalent as much as that of the former, are subjected to a neutralization reaction in a solvent such as ethanol or methanol at -50 to 80°C for 0.1 to 1 hour to obtain the compound (1).

The compound obtained by the above-mentioned neutralization reaction includes by-products such as the N-alkylglycolamide and an amine. The reaction product can be used as such according to a use, but may be purified through recrystallization, column chromatography, electrodialysis, extraction with a solvent, or the like if necessary to prepare a higher-purity product.

Step C

This step is a step wherein the N-alkylglycolamide (5) obtained in the step A is reacted with a sulfating agent, and then neutralized with a basic substance except for potassium-containing basic substances to obtain the compound represented by the aforementioned general formula (2) [hereinafter referred to briefly as the "compound (2)"].

The reaction of the N-alkylglycolamide (5) with the sulfating agent in this step can be effected in completely the same manner as in the step B.

Examples of the basic substance to be used in this step except for potassium-containing basic substances include hydroxides, carbonates and bicarbonates of alkali metals except for potassium and alkaline earth metals; ammonia; alkanolamines having 2 to 9 carbon atoms in total; alkylamines and alkenylamines having 1 to 22 carbon atoms in total;

pyridine substituted with alkyl or alkenyl having 1 to 18 carbon atoms; and basic amino acids. Specific examples of the basic substance include inorganic alkalis such as sodium hydroxide, lithium hydroxide, barium hydroxide, sodium hydrogencarbonate and sodium carbonate, ammonia, monoethanolamine, diethanolamine, triethanolamine, methylamine, dimethylamine, trimethylamine, ethylamine, diethylamine, triethylamine, decylamine, dodecylamine, tetradecylamine, hexadecylamine, octadecylamine, and basic amino acids, among which ammonia, sodium hydroxide and triethanolamine are preferred. They may be used in the form of an aqueous solution thereof or an alcoholic solution thereof for neutralization of the sulfuric ester. The degree of neutralization can be arbitrarily set. Further, the conditions of the neutralization reaction, the purification treatment, etc. may be the same as in the step B.

The detergent composition of the present invention comprises the compound (1) as the indispensable component, the content of which is preferably 1 to 70% by weight, further preferably 10 to 70% by weight.

Where the detergent composition of the present invention further comprises the compound (2) blended therein, the blending proportion thereof is preferably in the following range in terms of weight ratio:

compound (1)/compound (2) = 100/1 to 1/100.

In the detergent composition of the present invention, such an ingredient(s) as is used in conventional detergents such as shampoos and body detergents may further be used in combination besides the compound (1) and the compound (2) in so far as the effect of the present invention is not spoiled thereby. Examples of such an ingredient(s) for use in combination that may be appropriately blended include silicone derivatives or aqueous emulsions thereof as disclosed in Japanese Patent Laid-Open No. 43,433/1993; cationic surfactants as disclosed in Japanese Patent Laid-Open No. 172,133/1994; water-soluble cationic polymers; anionic surfactants; nonionic surfactants; ampholytic surfactants; dandruff removers; vitamin preparation; sterilizers; antiphlogistics; preservatives; humectants such as propylene glycol, glycerol, diethylene glycol monoethyl ether; sorbitol, and panthenol; colorants such as dyes and pigments; and other ingredients as disclosed in ENCYCLOPEDIA OF SHAMPOO INGREDIENTS (MICELLE PRESS, 1985).

The detergent composition of the present invention may further comprise a salt such as potassium chloride blended therein for the purpose of controlling the Krafft point thereof.

The detergent composition of the present invention can be prepared according to a customary method. For example, predetermined amounts of the compound (1), the compound (2), and an optional ingredient(s) as is used in conventional detergents such as shampoos and body detergents, provided that the effect of the present invention is not spoiled thereby, are weighed out, fed into equipment capable of agitation and mixture such as a blender vessel, and then mixed with each other while being heated or cooled to prepare a detergent composition. In this case, the heating temperature, the cooling temperature, the heating or cooling rate, the agitation speed, and the agitation time are not particularly limited. However, the pearly luster of the composition becomes more beautiful according to a method wherein they are once heated to a temperature of at least the Krafft point of the compound (1) and then cooled. Alternatively, a pearly aqueous dispersion of the compound (1) may be preliminarily prepared, and then mixed with other optional ingredient(s). Further alternatively, crystals of the compound (1) may be mixed with other optional ingredient(s).

The detergent composition of the present invention is preferably adjusted with a known acidic or alkaline agent to a pH of 3 to 10, especially preferably to a pH of 5 to 8.

The detergent composition of the present invention can be formed into a paste, a gel, or a liquid by using a viscosity modifier or the like according to a purpose. Further, it may comprise a cationic conditioning agent blended therein to be suitable as a hair detergent in the form of a rinse-in shampoo or the like.

The detergent composition of the present invention has an excellent detergency, excellent foaming properties, low irritating properties, a pearly luster, and an excellent resistance to hard water to be useful as a hair detergent, a body detergent, etc.

The detergent composition of the invention is inherently provided with a good luster of pearl even without incorporation of another substance to manifest a pearly luster.

[Examples]

The following Synthesis Examples of the compound (1) and the following Examples of the detergent composition according to the present invention will specifically illustrate the present invention, but should not be construed as limiting the scope of the present invention.

Additionally stated, % in Examples is by weight unless otherwise specified.

Synthesis Example 1

Synthesis of potassium salt of monolaurylglycolamide sulfate

(1) Synthesis of monolaurylglycolamide

A four-necked flask having a capacity of 1 liter and equipped with a dropping funnel, a stirrer and a thermometer was charged with 172.8 g (1.54 mol) of a 67.8% aqueous solution of glycolic acid, which was then stirred and heated in a nitrogen stream. The solution was heated up to 160°C while effecting dehydration, and then admixed with 300.0 g (1.62 mol) of laurylamine through the dropping funnel over about 1 hour. Thereafter, the resulting mixture was stirred at 160°C for 5 hours. The resulting reaction product was cooled to room temperature, and then dissolved in about 1.5 liters of ether. The resulting solution was transferred into a separatory funnel wherein it was then washed with 500 mℓ of ion-exchanged water three times. The ether phase was separated, and dried over anhydrous sodium sulfate. Ether was distilled off. The remaining solid was further dried under reduced pressure. Thus, there was obtained 348.1 g (yield: 92.9%) of monolaurylglycolamide, the hydroxyl number of which was 228.7 (theoretical value: 230.5).

On the other hand, values in the infrared absorption spectrum and $^1$H-NMR spectrum of the product were as follows.

Infrared Absorption Spectrum (KBr tablet method)

3334 $cm^{-1}$ (O-H stretching vibration),
3262 $cm^{-1}$ (N-H stretching vibration),
2920, 2854 $cm^{-1}$ (C-H stretching vibration),
1638 $cm^{-1}$ (C=O stretching vibration),

$^1$H-NMR Spectrum (δ, ppm) in $CDC1_3$

a: 0.89 ppm (t, 3H)
b: 1.30 ppm (m, 18H)
c: 1.55 ppm (m, 2H)
d: 3.27 ppm (q, 2H)
e: 7.02 ppm (b, 1H)
f: 4.02 ppm (s, 2H)
g: 5.10 ppm (b, 1H)

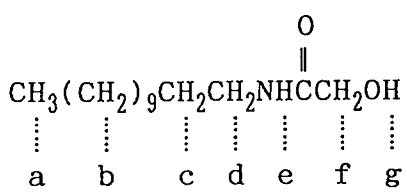

(2) Synthesis of potassium salt of monolaurylglycolamide sulfate

A four-necked flask having a capacity of 500 mℓ and equipped with a stirrer, a dropping funnel, a cooling tube and a thermometer was charged with 35.5 g (146 mmol) of monolaurylglycolamide synthesized in (1) above, which was then dissolved in 350 mℓ of chloroform. The resulting solution was stirred in a nitrogen stream at room temperature while dropwise adding thereto 17.9 g (153 mmol) of chlorosulfonic acid over about 30 minutes. In this step, the reaction system was cooled in a cool water bath in order to prevent the reaction temperature from exceeding 30°C. After the completion of the dropwise addition, the reaction system was stirred at 30°C for 1 hour. The reaction mixture was then poured into a mixed liquid composed of 150 g of cool water and 300 mℓ of n-butanol. The resulting mixture was transferred into a separatory funnel wherein the organic phase was then separated. This organic phase was neutralized with a 20% aqueous solution of caustic potash. After the neutralization, the solvent was distilled off under reduced pressure, and the residue was cooled to room temperature. Precipitated crystals were collected through filtration, and then dried under reduced pressure to obtain 47.0 g (yield: 89.0%) of a white powder of potassium salt of monolaurylglycolamide sulfate. The infrared absorption spectrum and $^1$H-NMR spectrum of the product were as follows. The purity of the product as an anionic surfactant was also measured by the Epton method to be 98.0%.

Infrared Absorption Spectrum (Kbr tablet method)

3346 $cm^{-1}$ (N-H stretching vibration),
2926, 2854 $cm^{-1}$ (C-H stretching vibration),
1659 $cm^{-1}$ (C=O stretching vibration),
1251, 1070 $cm^{-1}$ (S=O stretching vibration),

$^1$H-NMR Spectrum (δ, ppm) in $CDC1_3$

a: 0.57 ppm (t, 3H)
b: 0.97 ppm (m, 18H)
c: 1.21 ppm (m, 2H)
d: 2.90 ppm (q, 2H)
e: 7.02 ppm (b, 1H)
f: 4.15 ppm (s, 2H)

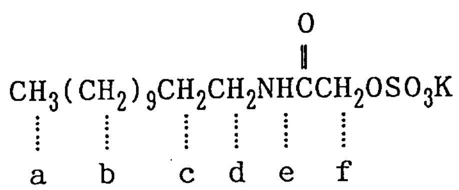

Example 1 and Comparative Examples 1 to 3

Lustrous shampoo compositions having respective recipes as shown in Table 1 were prepared, and evaluation test thereon were carried out according to the following methods. The results are shown in Table 1.

⟨Evaluation Test Methods⟩

20 womens were panelists. 20 g of a hair bundle (15 cm) of each panelist was coated with 1 g of each shampoo composition having a recipe as shown in Table 1, then washed, rinsed with running water of 40°C, and dried with a dryer to evaluate the easiness of foaming during hair washing, the state of foaming during hair washing, the slipperiness and passability between fingers of hair during hair washing, rinsing and drying, and the starchy feeling of hair during drying according to the following ratings.

Ratings

Easiness of foaming during hair washing

◎:Very easy to foam, ○: slightly easy to foam, △:slightly hard to foam, X: hard to foam.

State of foaming during hair washing

◎:very much foamed, ○: slightly much, △:a little, X: insufficient.

Slipperiness and passability between fingers during hair washing, rinsing and drying

◎:slippery, ○: slightly slippery, △:slightly squeaky, X: considerably squeaky.

Starchy feeling during hair drying

○:not starchy, △: slightly starchy, X:considerably starchy.

[Table 1]

| | | | Comp. Ex. 1 | Comp. Ex. 2 | Comp. Ex. 3 | Ex. 1 |
|---|---|---|---|---|---|---|
| Shampoo Recipe (%) | sodium salt of polyoxyethylene(3) laurylsulfate | | 20.0 | – | 10.0 | 10.0 |
| | potassium salt of monolaurylglycolamidesulfate | | – | – | – | 10.0 |
| | sodium monolaurylphosphate | | – | 15.0 | 10.0 | – |
| | distearyl ethylene glycol | | 0.3 | – | 0.3 | – |
| | sodium chloride | | – | 8.0 | – | – |
| | potassium chloride | | – | – | – | 1.0 |
| Results of Evaluation Tests | During Hair Washing | Easiness of Foaming | ○ | ◎ | ○ | ◎ |
| | | State of Foaming | ○ | ◎ | ○ | ◎ |
| | | Slipperiness/Passability between Fingers | ○ | ○ | ○ | ◎ |
| | During Rinsing | Slipperiness/Passability between Fingers | △ | × | × | ○ |
| | During Drying | Slipperiness/Passability between Fingers | △ | × | × | ○ |
| | | Starchy Feeling | △ | × | × | ○ |

Example 2

A shampoo having the following recipe was prepared. The shampoo obtained had a pearly appearance at room temperature, excellent foaming properties and an excellent detergency, and involved no squeaky feeling during rinsing and no starchy feeling during drying.

| | |
|---|---|
| sodium polyoxyethylene(3) laurylsulfate | 15.0% by weight |
| potassium salt of monolaurylglycolamide sulfate | 15.0 |
| lauroyldiethanolamide | 2.5 |
| potassium chloride | 1.5 |
| propylene glycol | 2.0 |
| citric acid | 3.0 |
| coloring matter | suitable amount |
| perfume | suitable amount |
| preservative | suitable amount |
| purified water | q.s. ad 100% by weight |

Example 3

A shampoo having the following recipe was prepared. The shampoo obtained had a pearly appearance at room temperature, excellent foaming properties and an excellent detergency, involved no squeaky feeling during rinsing, and

provided excellent slipperiness and passability between fingers of hair during drying.

| | |
|---|---|
| sodium monoethanollaurylamide polyoxyethylene(2) ether acetate | 12.0 wt. % |

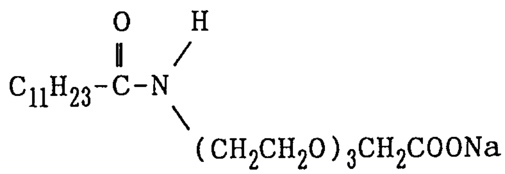

| | |
|---|---|
| potassium salt of monolaurylglycolamide sulfate | 12.0 |
| betaine laurylamidopropyldimethylaminoacetate | 3.0 |
| cationized cellulose*1 | 0.3 |
| dimethylpolysiloxane emulsion*2 | 3.5 |
| potassium chloride | 1.5 |
| propylene glycol | 2.0 |
| stearyltrimethylammonium chloride | 1.0 |
| citric acid | suitable amount |
| coloring matter | suitable amount |
| perfume | suitable amount |
| preservative | suitable amount |
| purified water | q.s. ad 100% by weight |

Note)

*1 Polymer JR400 manufactured by Union Carbide Japan K.K.

*2: BY22-835 manufactured by Dow Corning Toray Silicone Co., Ltd.

Example 4

A shampoo having the following recipe was prepared. The shampoo obtained had a pearly appearance at room temperature, excellent foaming properties and an excellent detergency, and involved no squeaky feeling during rinsing

and no starchy feeling during drying.

| | |
|---|---|
| sodium polyoxyethylene(3) laurylsulfate | 12.0% by weight |
| potassium salt of monolaurylglycolamide sulfate | 10.0 |
| sodium salt of monolaurylglycolamide sulfate | 2.0 |
| propylene glycol | 2.0 |
| hydroxyethylcellulose*1 | 0.1 |
| silicone emulsion*2 | 1.0 |
| (myristoyl-N-hydroxyethyl)aminoethyl-2-hydroxypropyltrimethylammonium chloride | 2.0 |
| citric acid | suitable amount |
| coloring matter | suitable amount |
| preservative | suitable amount |
| purified water | suitable amount |
| | total: 100% by weight |

Note)
*1: HEC-SE850 manufactured by Shin-Etsu Chemical Co., Ltd.
*2: SM-8702C manufactured by Dow Corning Toray Silicone Co., Ltd.

## Claims

**1.** A detergent composition comprising a compound represented by the general formula (1):

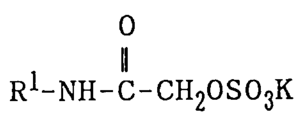

(1)

(wherein $R^1$ stands for a linear or branched alkyl or alkenyl group having 8 to 18 carbon atoms) and a carrier.

**2.** The composition as claimed in Claim 1, wherein $R^1$ is a linear or branched alkyl or alkenyl group having 10 to 14 carbon atoms.

**3.** The composition as claimed in Claim 1, wherein $R^1$ is a dodecyl group.

**4.** The composition as claimed in any one of Claims 1 to 3, which further comprises a compound represented by the general formula (2):

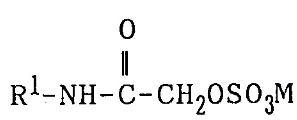

(2)

(wherein $R^1$ stands for a linear or branched alkyl or alkenyl group having 8 to 18 carbon atoms; and M stands for an alkali metal except for potassium, an alkaline earth metal, ammonium, an alkanolammonium, an alkylammonium, an alkenylammonium, an alkyl- or alkenyl-substituted pyridinium, or a basic amino acid).

**5.** The composition as claimed in Claim 4, wherein M of the general formula (2) is sodium, ammonium or triethanolammonium.

6. Use of the composition as defined in anyone of the claims 1 to 5 for deterging skin or hair.

7. Use of the composition as defined in anyone of the claims 1 to 5 as a manifester of a pearly luster.

European Patent Office

# EUROPEAN SEARCH REPORT

Application Number
EP 95 11 5971

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | EP-A-0 621 265 (KAO CORP) 26 October 1994<br>* the whole document *<br>--- | 1-6 | A61K7/50<br>C11D1/12 |
| A | US-A-2 255 082 (GENERAL ANALINE & FILM CORP.)<br>* the whole document *<br>--- | 1 | |
| A | US-A-2 120 512 (HENKEL & CIE)<br>* the whole document *<br>--- | 1 | |
| A | GB-A-499 022 (G. W. JOHNSON)<br>* the whole document *<br>----- | 1 | |

TECHNICAL FIELDS SEARCHED (Int.Cl.6)
A61K
C11D

The present search report has been drawn up for all claims

| Place of search | Date of completion of the search | Examiner |
|---|---|---|
| THE HAGUE | 6 February 1996 | Couckuyt, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)